# EUROPÄISCHE PATENTSCHRIFT

(11) **EP 4 530 472 B1**
(45) Veröffentlichungstag und Bekanntmachung des Hinweises auf die Patenterteilung: **18.03.2026**
(21) Anmeldenummer: 25156986.9
(22) Anmeldetag: 12.09.2022
(51) Int. Cl.: A61M 16/00, F04D 29/66, F04D 1/00, F04D 29/44

(54) **ATEMTHERAPIEGERÄT UND LÜFTEREINRICHTUNG**
RESPIRATORY THERAPY DEVICE AND FAN DEVICE
APPAREIL DE THÉRAPIE RESPIRATOIRE ET DISPOSITIF VENTILATEUR

(30) Priorität: 28.09.2021 DE 102021004890
(43) Veröffentlichungstag der Anmeldung: 02.04.2025
(62) Teilanmeldung aus: 22195097.5
(73) Patentinhaber: Löwenstein Medical Technology S.A., 2557 Luxembourg (LU)
(72) Erfinder: GERLACH, Angela, 22549 Hamburg (DE)
(74) Vertreter: Löwenstein Medical IP

(56) Entgegenhaltungen:
- EP-A1- 0 573 895
- EP-A2- 0 872 643
- WO-A1-2011/062633
- WO-A1-2013/009193
- WO-A1-2013/072846
- WO-A1-2020/070349
- WO-A1-2020/243958
- AU-A1- 2020 233 744
- DE-U1- 212020 000 550
- US-A1- 2012 180 792
- US-A1- 2015 219 119
- US-A1- 2021 170 125
- US-A1- 2021 220 585

## Beschreibung

Beatmungs- und Atemtherapiegeräte zur Beatmung bzw. Atmungsunterstützung oder zur Hustenunterstützung weisen für die Durchführung einer Atemtherapie eine Lüftereinrichtung zum Erzeugen einer Atemluftströmung auf. Im Gehäuse der Lüftereinrichtung ist üblicherweise wenigstens ein drehbar gelagertes Lüfterrad mit einer Mehrzahl von Schaufelelementen angeordnet.

Damit die Behandlung bzw. Therapie nicht als störend empfunden wird, sollten die Betriebsgeräusche der Lüftereinrichtung möglichst gering sein. Für einen effizienten Druckaufbau und eine gezielte Atemtherapie ist es zudem entscheidend, dass hohe Drehzahlen erreicht werden und möglichst zügige und kurzfristige Drehzahlanpassungen des Lüfterrades erfolgen können.

Durch Wirbelablösungen und turbulente An- und Abströmungen innerhalb des Gehäuses entstehen Strömungsgeräusche. Diese Strömungsgeräusche setzen sich aus einem breitbandigen Rauschanteil und überlagerten Tönen zusammen. Der Drehklang wird insbesondere durch die Laufradschaufeln verursacht, wenn das Fluid aus den Schaufelkanälen auf die Gehäusezunge des Gehäuses strömt.

US2015/219119A1, US2021/220585A1, EP0573895A1, US 2021/170125A1 und AU2020233744A1 offenbaren Gebläse mit Vorrichtungen zur Schallreduzierung.

Aufgabe der vorliegenden Erfindung ist, ein Atemtherapiegerät zur Verfügung zu stellen, welches die zuvor genannten Anforderungen möglichst vorteilhaft erfüllen kann.

Diese Aufgabe wird gelöst mit einem Atemtherapiegerät des Anspruchs 1 sowie mit einer Lüftereinrichtung gemäß Anspruch 17. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Das erfindungsgemäße Atemtherapiegerät umfasst wenigstens eine Lüftereinrichtung zum Erzeugen einer Atemluftströmung für die Durchführung einer Atemtherapie, wobei die Lüftereinrichtung ein Gehäuse und wenigstens ein in dem Gehäuse drehbar gelagertes Lüfterrad umfasst, wobei die Atemluft durch einen innerhalb des Gehäuses ausgebildeten Kanal transportiert wird, wobei das Gehäuse wenigstens ein Strukturelement aufweist, das die Schallemission verringert.

In manchen Ausführungsformen ist das Atemtherapiegerät dadurch gekennzeichnet, dass das Gehäuse mindestens eine Innenseite 16i, 17i umfasst und der Kanal ein Lumen umfasst, wobei das Lumen des Kanals von der Innenseite 16i und/oder 17i umschlossen ist.

In manchen Ausführungsformen ist das Atemtherapiegerät dadurch gekennzeichnet, dass das mindestens eine Strukturelement im Lumen des Kanals angeordnet ist.

In manchen Ausführungsformen ist das Atemtherapiegerät dadurch gekennzeichnet, dass das mindestens eine Strukturelement in und/oder an der Innenseite 16i, 17i angeordnet ist.

In manchen Ausführungsformen ist das Atemtherapiegerät dadurch gekennzeichnet, dass der Kanal einen Einlasskanal, mindestens einen Strömungskanal und mindestens einen Auslasskanal umfasst, die kommunizierend miteinander in Verbindung stehen, wobei der Kanal einen Strömungsweg mit einer Strömungsrichtung ausbildet.

In manchen Ausführungsformen ist das Atemtherapiegerät dadurch gekennzeichnet, dass die Strömungsrichtung vom Einlasskanal zum Strömungskanal zum Auslasskanal verläuft.

In manchen Ausführungsformen ist das Atemtherapiegerät dadurch gekennzeichnet, dass das Strukturelement als mindestens eine Trennwand und/oder mindestens eine Senkbohrung ausgebildet ist.

In manchen Ausführungsformen ist das Atemtherapiegerät dadurch gekennzeichnet, dass die Trennwand zumindest abschnittsweise im Lumen des Strömungskanals und/oder im Lumen des Auslasskanals angeordnet ist.

In manchen Ausführungsformen ist das Atemtherapiegerät dadurch gekennzeichnet, dass die Trennwand den Strömungskanal und/oder den Auslasskanal zumindest abschnittsweise zumindest teilweise in mindestens zwei Kanäle unterteilt.

In manchen Ausführungsformen ist das Atemtherapiegerät dadurch gekennzeichnet, dass die Trennwand derart im Kanal angeordnet ist, dass eine zweite Zunge im Strömungskanal oder im Auslasskanal ausgebildet ist.

In manchen Ausführungsformen ist das Atemtherapiegerät dadurch gekennzeichnet, dass die mindestens eine Senkbohrung in der Innenseite 16i und/oder 17i im Bereich des Strömungskanals und/oder des Auslasskanals angeordnet ist.

In manchen Ausführungsformen ist das Atemtherapiegerät dadurch gekennzeichnet, dass die Senkbohrungen durch eine Vertiefung in der Innenseite 16i und/oder 17i ausgebildet sind.

In manchen Ausführungsformen ist das Atemtherapiegerät dadurch gekennzeichnet, dass die Senkbohrungen eine Tiefe von 0,5 mm bis 2,5 mm, bevorzugt von 1 mm bis 2 mm, besonders bevorzugt von 1,4 mm aufweisen.

In manchen Ausführungsformen ist das Atemtherapiegerät dadurch gekennzeichnet, dass die Senkbohrungen rund und/oder oval ausgebildet sind.

In manchen Ausführungsformen ist das Atemtherapiegerät dadurch gekennzeichnet, dass die Senkbohrungen einen Durchmesser von 1 mm bis 7 mm aufweisen, bevorzugt von 2 mm bis 5 mm, besonders bevorzugt von 4 mm aufweisen.

In manchen Ausführungsformen ist das Atemtherapiegerät dadurch gekennzeichnet, dass die Innenseiten 16i und/oder 17i 1 bis 100 Senkbohrungen aufweisen, bevorzugt bis 1 bis 50, besonders bevorzugt bis 4 bis 20.

Die erfindungsgemäße Lüftereinrichtung ist für ein Atemtherapiegerät vorgesehen, wie es vorzugsweise zuvor beschrieben wurde. Die Lüftereinrichtung umfasst ein Gehäuse und wenigstens ein in dem Gehäuse drehbar gelagertes Lüfterrad. Dabei ist die Lüftereinrichtung vorzugsweise wie zuvor für das erfindungsgemäße Atemtherapiegerät beschrieben ausgebildet.

In den Figuren sind Ausführungsbeispiele des erfindungsgemäßen Atemtherapiegeräts dargestellt. Es zeigen:
**Fig. 1** eine schematische Darstellung eines erfindungsgemäßen Atemtherapiegerätes in einer perspektivischen Ansicht
**Fig. 2** eine schematische Außenansicht eines erfindungsgemäßen Gehäuses von vorne
**Fig. 3** eine schematische Außenansicht eines erfindungsgemäßen Gehäuses von hinten
**Fig. 4** einen Längsschnitt durch ein erfindungsgemäßes Gehäuse eines Ausführungsbeispiels mit einer Innenansicht des Hinterteils, wobei **Fig. 4A** eine Ansicht mit eingesetztem Lüfterrad und ohne Trennwand zeigt und **Fig. 4B** eine Ansicht ohne eingesetztes Lüfterrad und mit einer erfindungsgemäßen Trennwand zeigt.
**Fig. 5** einen Längsschnitt durch das Gehäuse aus Fig.4 mit einer Innenansicht des Vorderteils
**Fig. 6** einen vergrößerten Ausschnitt aus Fig. 4B
**Fig. 7** eine schematische Außenansicht des Gehäuses aus Fig. 4-6 von der Seite mit Blickrichtung in einen Druckstutzen hinein
**Fig. 8** einen Querschnitt durch das Gehäuse aus Fig. 4-7
**Fig. 9** einen Längsschnitt durch ein erfindungsgemäßes Gehäuse eines weiteren Ausführungsbeispiels mit einer Innenansicht des Hinterteils mit eingesetztem Lüfterrad
**Fig. 10** einen Längsschnitt durch das Gehäuse des weiteren Ausführungsbeispiels mit einer Innenansicht des Vorderteils
**Fig. 11** eine schematische Außenansicht des Gehäuses entsprechend des weiteren Ausführungsbeispiels aus Fig. 9-10 von der Seite mit Blickrichtung in einen Druckstutzen hinein
**Fig. 12** einen Querschnitt durch das Gehäuse des weiteren Ausführungsbeispiels aus Fig.9-11
**Fig. 13** einen Längsschnitt durch ein erfindungsgemäßes Gehäuse eines alternativen Ausführungsbeispiels mit einer Innenansicht des Hinterteils mit eingesetztem Lüfterrad
**Fig. 14** einen Querschnitt durch das Gehäuse des alternativen Ausführungsbeispiels aus Fig. 13.
**Figur 15** schematisch einen Querschnitt durch die Gehäusewand einer Innenseite des Vorderteils bzw. einer Innenseite des Hinterteils des alternativen Ausführungsbeispiels aus Fig. 13/14.

### Ausführungsbeispiele

In den nachfolgenden Ausführungsbeispielen ist ein erfindungsgemäßes Atemtherapiegerät 70 gezeigt. Weitere Merkmale und Vorteile der vorliegenden Erfindung werden in den nachfolgenden Beschreibungen von Ausführungsbeispielen anhand der Figuren deutlich. Die Erfindung ist nicht auf die dargestellten Ausführungsbeispiele beschränkt.

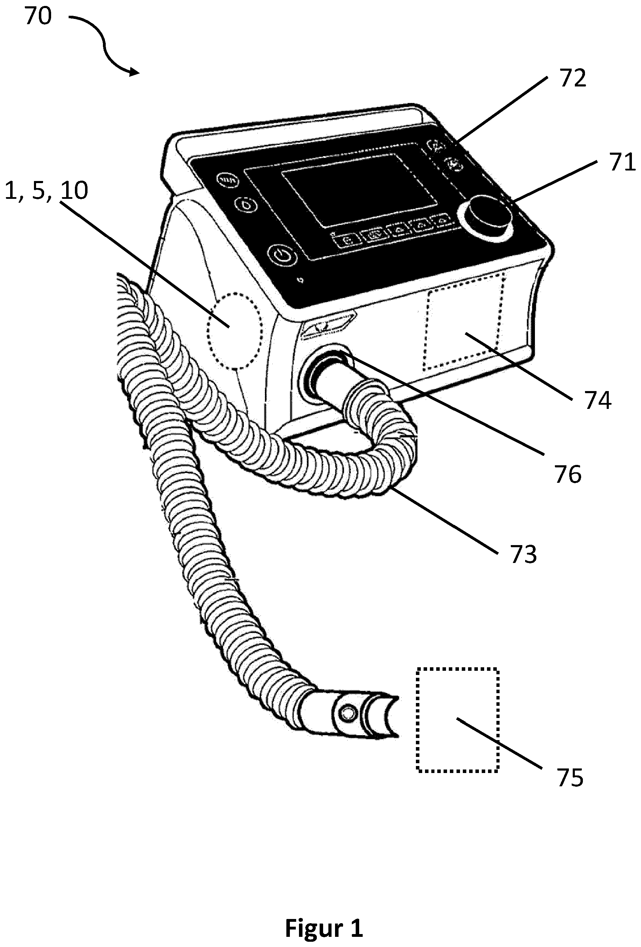

**Figur 1** zeigt eine schematische Darstellung eines erfindungsgemäßen Atemtherapiegerätes 70 in einer perspektivischen Ansicht.

Das erfindungsgemäße Atemtherapiegerät 70 ist beispielsweise ein Beatmungsgerät für klinische oder Heimanwendungen, ein Notfallbeatmungsgerät, ein Atemtherapiegerät oder ein Hustentherapiegerät. Das Atemtherapiegerät 70 ist mit einer hier nicht sichtbar im Geräteinneren untergebrachten erfindungsgemäßen Lüftereinrichtung 1 ausgestattet, mit der eine Atemluftströmung für eine Atemtherapie erzeugt wird.

Die Lüftereinrichtung 1 hat ein Gehäuse 10, in dem ein Lüfterrad 5 angeordnet ist. Das Lüfterrad 5 ist in dem Gehäuse 10 drehbar gelagert angeordnet. Das Lüfterrad 5 umfasst mehrere Schaufelelemente 6. Zum Drehen des Lüfterrades 5 weist die Lüftereinrichtung 1 einen elektrischen Antrieb auf, der Rotationsenergie auf das Lüfterrad 5 überträgt. Durch die Rotationsbewegung entsteht am Lüfterrad 5 eine Druck- und eine Unterdruckseite, wodurch die Atemluftströmung entsteht. Atemluft umfasst im Sinne der Erfindung jegliches Fluid, Atemgas und/oder Gasgemisch, welches sich zur Beatmung, Atmung und/oder Atemtherapie eignet und eingesetzt werden kann. Insbesondere kann die Atemluft auch Sauerstoff sein oder mit Sauerstoff angereicherte Luft sein.

Lüftereinrichtungen 1, die in Atemtherapiegeräten 70 eingesetzt werden, sind üblicherweise Radialgebläse. Bei Radialgebläsen tritt ein Fluid, beispielsweise Atemluft, in Achsrichtung in die Lüftereinrichtung 1 ein und tritt senkrecht zur Achsrichtung wieder aus der Lüftereinrichtung 1 heraus. Die Lüftereinrichtung 1 kann neben Atemluft auch jedes andere Gasgemisch transportieren, das für eine Atemtherapie benötigt wird.

Die Lüftereinrichtung 1 wird über eine nicht sichtbar im Geräteinneren angeordnete Steuereinrichtung 74 angesteuert. Beispielsweise stellt die Steuereinrichtung 74 in Abhängigkeit der Therapievorgaben eine bestimmte Drehzahl des Lüfterrades 5 ein bzw. regelt die Lüfterdrehzahl auf einen Sollwert.

Das Atemtherapiegerät 70 ist hier mit einer Bedieneinrichtung 71 und mit einer Anzeigeeinrichtung 72 ausgestattet. Dabei erfolgt hier beispielsweise ein Teil der Bedienung über eine berührungsempfindliche Oberfläche der Anzeigeeinrichtung 72.

Das Atemtherapiegerät 70 weist eine Schnittstelle 76 zum Koppeln eines Schlauchsystems 73 zur Beatmung bzw. Hustenunterstützung auf. Die mittels der Lüftereinrichtung 1 erzeugte Atemluftströmung wird über das Schlauchsystem 73 dem nicht gezeigten Patienten zugeführt. An das Schlauchsystem 73 kann ein hier nicht näher dargestelltes Patienteninterface 75 wie beispielsweise eine Atemmaske angeschlossen werden.

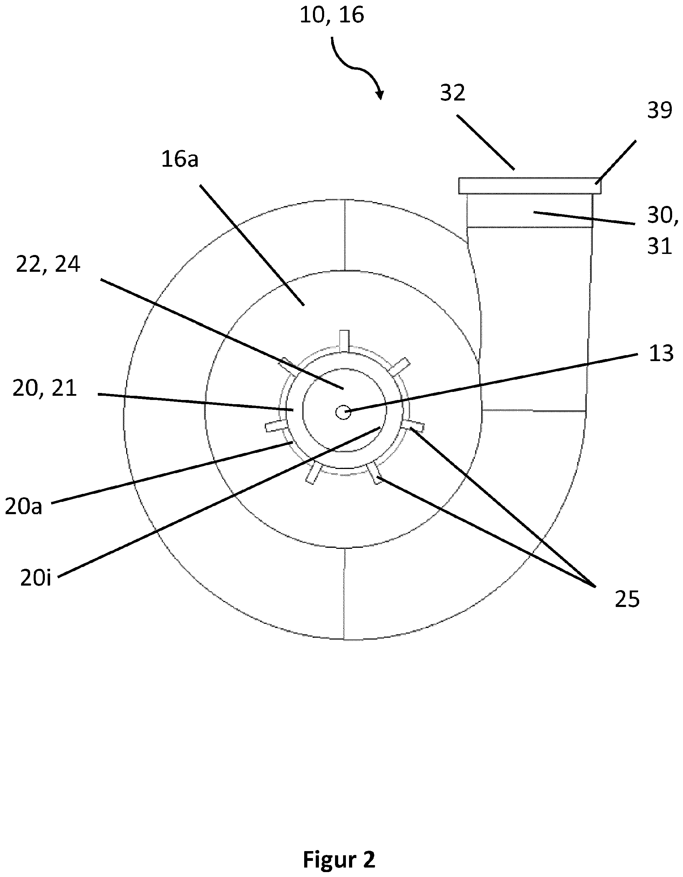

Die Figuren 2 bis 14 zeigen verschiedene Ausführungsbeispiele der erfindungsgemäßen Lüftereinrichtung 1, wobei das Gehäuse 10 der Lüftereinrichtung 1 in unterschiedlichen Ausführungsbeispielen und in unterschiedlichen Ansichten exemplarisch dargestellt ist. Die Erfindung ist nicht auf die dargestellten Ausführungsbeispiele beschränkt.

Das Gehäuse 10 der Lüftereinrichtung 1 ist in der Regel spiralförmig. Die Lüftereinrichtung 1 umfasst einen Saugstutzen 20 und einen Druckstutzen 30. Über den Saugstutzen 20 kann das Fluid, beispielsweise Atemluft angesaugt werden. Über den Druckstutzen 30 kann das Fluid abgegeben werden. Der Saugstutzen weist eine Mittelachse 13 auf. Durch den Saugstutzen 20 tritt Atemluft in Achsrichtung in die Lüftereinrichtung 1 ein. Durch den Druckstutzen 30 tritt die Atemluft senkrecht zur Achsrichtung wieder aus der Lüftereinrichtung 1 heraus.

Das spiralförmige Gehäuse 10 sorgt dafür, dass die Atemluft im Gehäuse 10 gesammelt und zum Druckstutzen 30 geleitet wird, wo sie aus dem Gehäuse 10 austritt. Dabei wird verhindert, dass kreisende Austrittsströmungen entstehen, die zu Verlusten führen. Gleichzeitig wird durch das Spiralgehäuse 10 ein Teil der Geschwindigkeitsenergie der Atemluft in Druckenergie umgewandelt.

Das spiralförmige Gehäuse 10 hat eine Gehäusewand 11 und umfasst ein Vorderteil 16 und ein Hinterteil 17. Das Vorderteil 16 ist per definitionem das Element, das den Saugstutzen 20 umfasst. Das Hinterteil 17 ist per definitionem das Element, das eine Öffnung 40 umfasst, durch die eine Welle eines Elektromotors mit dem Lüfterrad 5 verbunden werden kann (nicht gezeigt).

Vorderteil 16 und Hinterteil 17 können einstückig oder zweistückig gefertigt sein. Bevorzugt werden Vorderteil 16 und Hinterteil 17 zweistückig gefertigt. Eine zweistückige Fertigung erleichtert die Fertigung und Montage. Die Lüftereinrichtung 1 befindet sich in einem Anwendungszustand, wenn Vorderteil 16 und Hinterteil 17 miteinander verbunden sind und ein nicht gezeigtes Lüfterrad 5 derart umschließen, dass das Lüfterrad 5 drehbar gelagert ist.

Vorderteil 16 und Hinterteil 17 können beispielsweise miteinander verklebt, verschweißt, verschraubt, verklemmt, verpresst oder ähnliches werden. Vorderteil 16 und/oder Hinterteil 17 können beispielsweise auch mit einem Hinterschnitt miteinander verbunden werden. Auf diese Weise können Vorderteil 16 und Hinterteil 17 miteinander verbunden werden. Die Verbindung kann reversibel oder irreversibel sein. Durch die Verbindung bei einer zweistückigen Fertigung kann eine Verbindungsfuge 18 entstehen. Die Verbindungsfuge 18 ist bevorzugt derart gering ausgeprägt, dass sie keine Beeinträchtigung des Strömungsweges verursacht.

Das Gehäuse 10 kann aus unterschiedlichen Materialien hergestellt werden. Die Wahl des Materials kann Auswirkungen auf die Formtreue des Gehäuses 10 und die Rauigkeit der Oberflächen haben. Das Gehäuse 10 kann beispielsweise aus Kunststoff und/oder Metall gefertigt sein. Beispielsweise ist das Gehäuse 10 aus einem spritzbaren Kunststoff gefertigt. Bevorzugt ist das Gehäuse 10 aus Polycarbonat und/oder Polyamid gefertigt. Beispielsweise ist das Gehäuse 10 aus Polyamid PA12 hergestellt.

**Figur 2** zeigt eine schematische Außenansicht eines erfindungsgemäßen Gehäuses 10 von vorne. Fig. 2 zeigt das Vorderteil 16 des Gehäuses 10. Das Vorderteil 16 umfasst eine Außenseite 16a. Die Außenseite 16a weist in der Regel eine im Wesentlichen glatte Oberfläche auf.

Das Vorderteil 16 umfasst den Saugstutzen 20. Der Saugstutzen 20 hat eine Saugstutzenwand 21 mit einer Saugstutzen-Außenseite 20a und einer Saugstutzen-Innenseite 20i. Der Saugstutzen 20 bildet einen Einlasskanal 24 und umfasst eine Einlassöffnung 22. Der Saugstutzen 20 kann als länglicher Hohlkörper ausgebildet sein, in dessen Lumen der Einlasskanal 24 verläuft. Die Saugstutzen-Innenseite 20i umfasst das Lumen des Saugstutzens 20, in dem der Einlasskanal 24 verläuft.

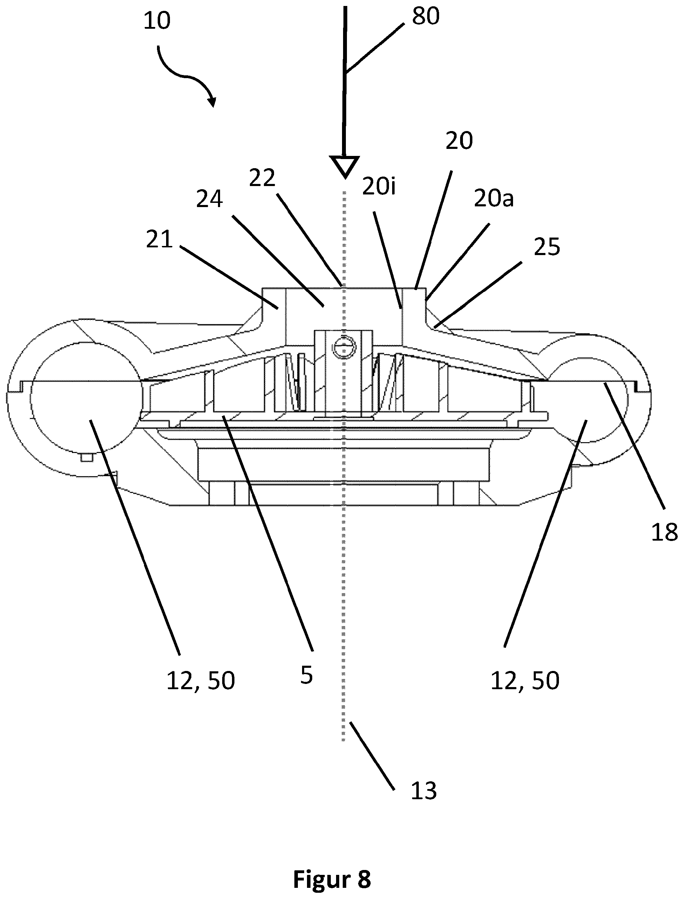

Der Durchmesser des Saugstutzens 20 kann jegliche geeignete Geometrie aufweisen. In der Regel ist der Durchmesser des Saugstutzens 20 rund ausgebildet, beispielsweise kreisrund. Der Saugstutzen 20 ragt üblicherweise aus der Außenkontur des Gehäuses 10 hervor und tritt aus der Ebene der Außenseite 16a heraus (siehe bspw. Fig. 8).

Durch den Einlasskanal 24 im Saugstutzen 20 kann ein Fluid, beispielsweise ein Atemgas oder Gasgemisch, in das Gehäuse 10 eintreten. Bevorzugt kann Atemluft über den Einlasskanal 24 und den Saugstutzen 20 in das Gehäuse 10 eintreten. Über den Saugstutzen 20 kann beispielsweise Umgebungsluft angesaugt werden. In manchen Ausführungsformen kann der Saugstutzen 20 mit einem hier nicht gezeigten Schlauch verbunden sein, über den ein definiertes Gas und/oder Gasgemisch in den Einlasskanal 24 eingeleitet werden kann.

Um eine Schlauchverbindung zum Saugstutzen 20 zu ermöglichen, kann der Saugstutzen 20 einen Saugstutzen-Flansch 29 umfassen. Der Saugstutzen-Flansch 29 kann als ringförmige Verbreiterung am Ende des Saugstutzens ausgebildet sein (nicht gezeigt).

Die Saugstutzen-Innenseite 20i kann beispielsweise glatt ausgebildet sein. In manchen Ausführungsformen ist es auch denkbar, dass die Saugstutzen-Innenseite 20i strukturiert ausgebildet ist und beispielsweise Rippen, Furchen, Kanäle oder dergleichen aufweist, um die Strömungseigenschaften des Fluids zu optimieren (nicht gezeigt).

Um den Saugstutzen 20 herum kann mindestens eine, bevorzugt mehrere Stützrippen 25 angeordnet sein. Die Stützrippen 25 können beispielsweise zumindest abschnittsweise auf der Saugstutzen-Außenseite 20a angeordnet sein. Beispielsweise können die Stützrippen 25 an der Saugstutzen-Außenseite 20a und an der Außenseite des Gehäusevorderteils 16a angeordnet sein und diese miteinander stützend verbinden. Die Stützrippen 25 sind eingerichtet und ausgebildet, dem Saugstutzen 20 Stabilität zu verleihen.

Das Gehäuse 10 umfasst den Druckstutzen 30. Der Druckstutzen 30 weist eine Auslassöffnung 32 auf. Der Auslassöffnung 32 stellt eine druckseitige Ausströmöffnung aus dem Gehäuse 10 dar. Durch den Druckstutzen 30 kann ein Fluid, beispielsweise ein Atemgas oder Gasgemisch, aus dem Gehäuse 10 austreten. Bevorzugt kann Atemluft durch den Druckstutzen 30 austreten.

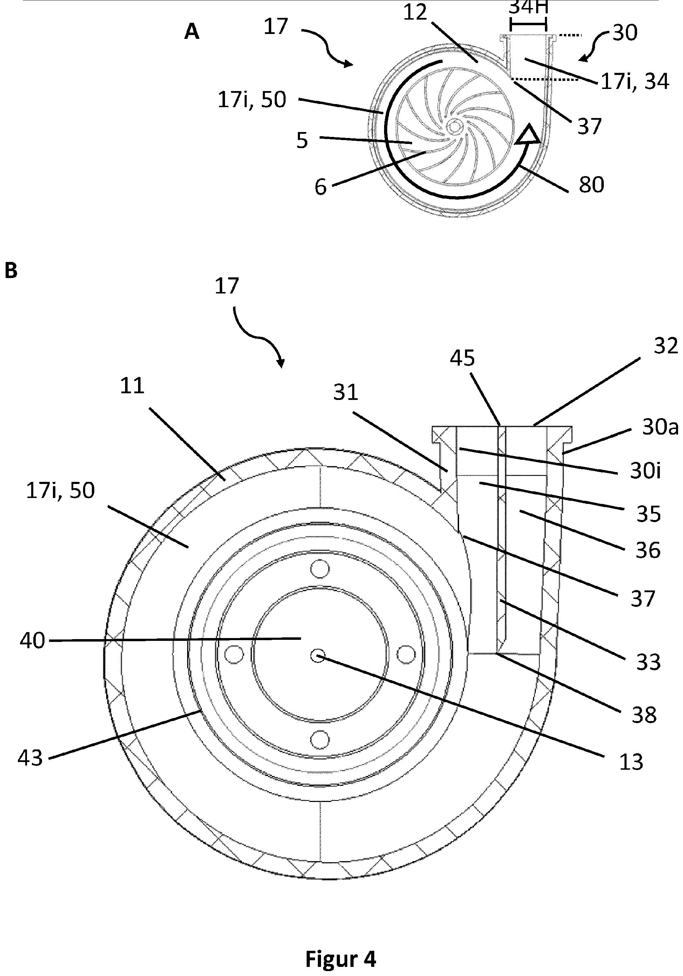

Der Druckstutzen 30 weist eine Druckstutzenwand 31 mit einer Druckstutzen-Außenseite 30a und einer Druckstutzen-Innenseite 30i (siehe bspw. Fig. 4) auf und ist in der Regel zumindest teilweise als länglicher Hohlkörper ausgebildet. Der Hohlkörper des Druckstutzens 30 bildet einen Auslasskanal 34 (siehe bspw. Fig. 4). Der Durchmesser des Druckstutzens 30 kann jegliche geeignete Geometrie aufweisen. In der Regel ist der Durchmesser des Druckstutzens 30 rund ausgebildet, beispielsweise kreisrund.

Der Druckstutzen 30 kann mit einem hier nicht gezeigten Schlauch oder Schlauchsystem 73 verbunden sein, über den die Atemluft aus dem Gehäuse 10 abgeführt wird. Über das Schlauchsystem 73 kann die Atemluft dem nicht dargestellten Patienten zugeführt werden. Um eine Schlauchverbindung zum Druckstutzen 30 zu ermöglichen, kann der Druckstutzen 30 einen Druckstutzen-Flansch 39 umfassen. Der Druckstutzen-Flansch 39 kann als ringförmige Verbreiterung am Ende des Druckstutzens ausgebildet sein. Der Druckstutzen-Flansch 39 kann beispielsweise an die Druckstutzenwand 31 angeformt sein.

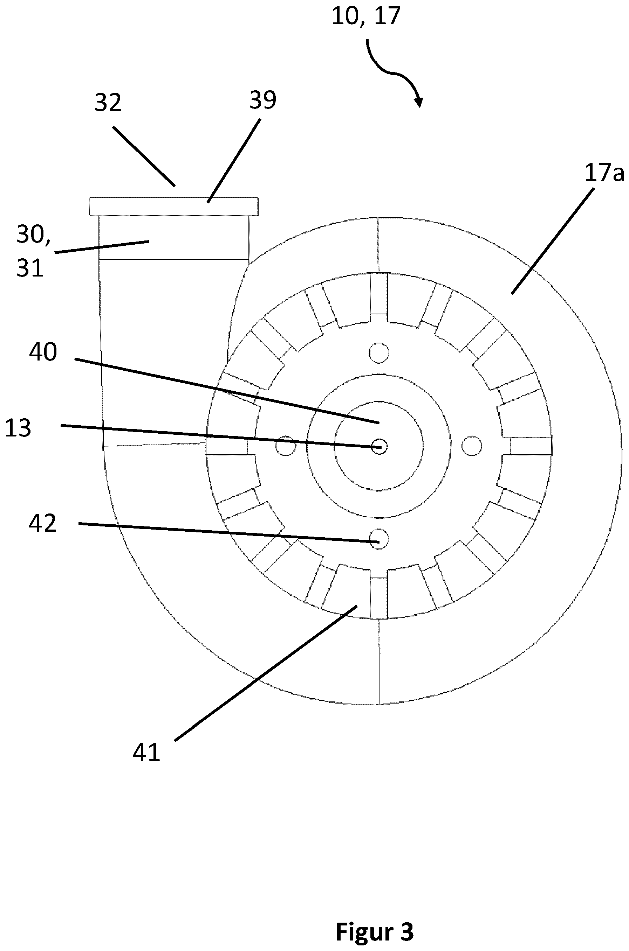

**Figur 3** eine schematische Außenansicht eines erfindungsgemäßen Gehäuses 10 von hinten. Fig. 3 zeigt das Hinterteil 17 des Gehäuses 10. Das Hinterteil 17 umfasst eine Außenseite 17a. Die Außenseite 17a weist in der Regel eine im Wesentlichen glatte Oberfläche auf.

Das Hinterteil 17 umfasst mindestens eine Öffnung 40. Durch die Öffnung 40 kann eine Welle eines Elektromotors hindurchführen, die im Inneren des Gehäuses 10 mit einem Lüfterrad 5 verbunden werden kann, um dieses anzutreiben (nicht gezeigt). Die Öffnung 40 kann beispielsweise rund ausgebildet sein. In alternativen Ausführungsformen kann die Öffnung 40 auch jegliche andere geeignete Geometrie aufweisen.

Gehäuse 10 und Elektromotor können beispielsweise miteinander verschraubt werden. In einer bevorzugten Ausführungsform kann das Gehäuse 10 auf den Elektromotor gespritzt werden.

Das Hinterteil 17 kann ein oder mehrere Bohrungen 42 aufweisen. Beispielsweise kann das Hinterteil 17 vier Bohrungen 42 aufweisen. Die Bohrungen 42 sind bevorzugt radial um die Öffnung 40 herum angeordnet. Über die Bohrungen 42 kann das Gehäuse 10 mit dem Elektromotor verschraubt werden. Radial um die Öffnung 40 herum können zur Versteifung ein oder mehrere Elemente 41 angeordnet sein.

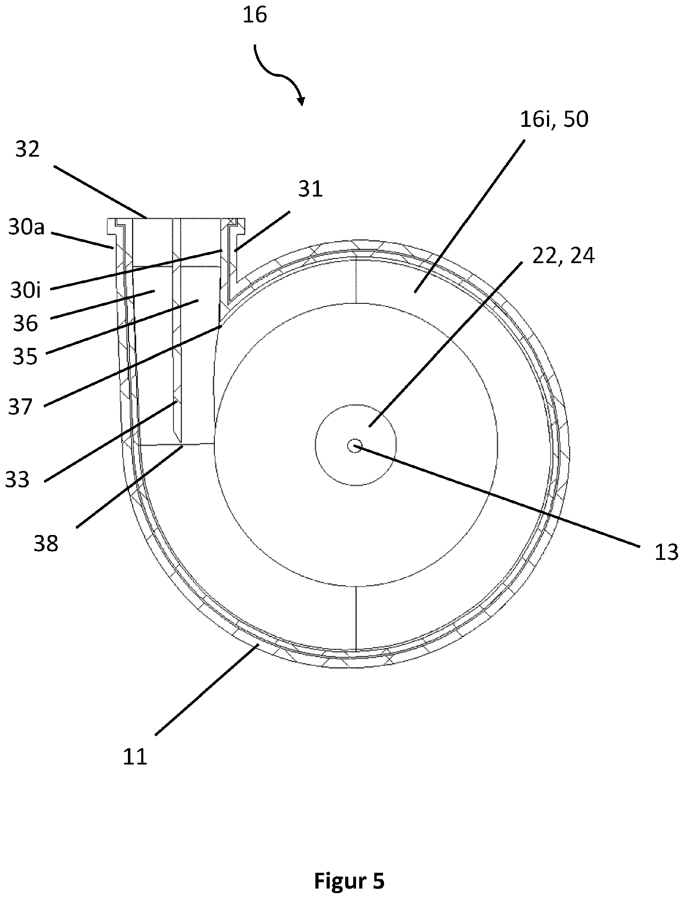

**Figur 4** zeigt einen Längsschnitt durch ein erfindungsgemäßes Gehäuse 10 eines Ausführungsbeispiels mit einer Innenansicht des Hinterteils 17, wobei **Fig. 4A** eine Ansicht mit eingesetztem Lüfterrad 5 und ohne Trennwand 33 zeigt und **Fig. 4B** eine Ansicht ohne eingesetztes Lüfterrad 5 und mit einer erfindungsgemäßen Trennwand 33 zeigt. **Figur 5** zeigt einen Längsschnitt durch das Gehäuse 10 aus Fig. 4 mit einer Innenansicht des Vorderteils 16.

Aus Fig. 4B wird ersichtlich, dass das Hinterteil 17 eine Aufnahmeeinrichtung 43 umfassen kann, die das Lüfterrad 5 aufnehmen kann. In Fig. 4A ist beispielhaft dargestellt, wie das Lüfterrad 5 zur Herstellung eines Anwendungszustandes positioniert sein kann. Die Lüftereinrichtung 1 befindet sich in einem Anwendungszustand, wenn das Lüfterrad 5 in die Aufnahmeeinrichtung 43 eingesetzt ist und Vorderteil 16 und Hinterteil 17 miteinander verbunden sind. Dabei ist das Lüfterrad 5 derart eingesetzt, dass das Lüfterrad 5 drehbar gelagert ist.

In der Regel ist innerhalb der Aufnahmeeinrichtung 43 die Öffnung 40 angeordnet, durch die die Welle eines Elektromotors hindurchführt, der das Lüfterrad 5 antreibt. Die Mittelachse 13 kann in der Mitte der Öffnung 40 liegen.

Das Hinterteil 17 umfasst eine Innenseite 17i (Fig. 4). Die Oberfläche der Innenseite 17i kann glatt oder zumindest teilweise strukturiert ausgebildet sein. In der Regel weist die Innenseite 17i eine im Wesentlichen glatte Oberfläche auf.

Das Vorderteil 16 umfasst eine Innenseite 16i (Fig. 5). Die Oberfläche der Innenseite 16i kann glatt oder zumindest teilweise strukturiert ausgebildet sein. In der Regel weist die Innenseite 16i eine im Wesentlichen glatte Oberfläche auf.

In einem Anwendungszustand, also wenn Vorderteil 16 und Hinterteil 17 miteinander verbunden sind, ist im Inneren des Gehäuses 10 ein Kanal 12 eingerichtet und ausgebildet. Durch den Kanal 12 kann das Fluid, beispielsweise Atemluft strömen. Der Kanal 12 ist als Hohlkörper ausgebildet. Der Kanal 12 hat ein Lumen. Das Lumen des Kanals 12 wird zumindest teilweise von den Innenseiten 16i und/oder 17i umgeben.

Die Innenseiten 16i, 17i können gewölbt sein, um den Kanal 12 auszubilden. Beispielsweise können die Innenseiten 16i, 17i derart gewölbt sein, dass der Kanal einen im Wesentlichen runden Kanal 12 ausbilden. Beispielsweise weist der Kanal 12 einen im Wesentlichen kreisrunden Querschnitt auf (siehe beispielsweise Fig. 8).

Die Druckstutzen-Innenseite 30i ist ein Teilabschnitt der Innenseiten 16i und 17i. Die Innenseiten 16i und 17i, die das Lumen des Druckstutzens 30 umschließen, werden hierin auch als Druckstutzen-Innenseite 30i bezeichnet.

Der Kanal 12 kann einen Einlasskanal 24 (siehe Fig. 2), einen Strömungskanal 50 und einen Auslasskanal 34 umfassen (siehe Fig. 4A). Einlasskanal 24, Strömungskanal 50 und Auslasskanal 34 sind kommunizierende Kanäle. Einlasskanal 24, Strömungskanal 50 und Auslasskanal 34 stehen fluidisch miteinander in Verbindung. Einlasskanal 24, Strömungskanal 50 und Auslasskanal 34 bilden einen Strömungsweg mit einer Strömungsrichtung 80 aus (siehe Fig. 4A). Beispielsweise stehen Einlasskanal 24, Strömungskanal 50 und Auslasskanal 34 pneumatisch miteinander in Verbindung. Ein Fluid, beispielsweise Atemluft, kann in den Einlasskanal 24 strömen und von dort über das Lüfterrad 5 in den Strömungskanal 50 und vom Strömungskanal 50 in den Auslasskanal 34 strömen. Die Strömungsrichtung 80 verläuft in der Regel vom Einlasskanal 24 über den Strömungskanal 50 in den Auslasskanal 34.

Bei einer zweistückigen Fertigung der Lüftereinrichtung 1 kann der Strömungskanal 50 und/oder der Auslasskanal 34 von der Innenseite des Vorderteils 16i und von der Innenseite des Hinterteils 17i umschlossen sein.

In einem Anwendungszustand umschließen die Innenseiten 16i, 17i zumindest teilweise zumindest abschnittsweise den Strömungskanal 50. In einem Anwendungszustand umschließt die Druckstutzen-Innenseite 30i zumindest teilweise zumindest abschnittsweise den Auslasskanal 34. Der Auslasskanal 34 durchzieht den Druckstutzen 30 der Länge nach.

Die Druckstutzen-Innenseite 30i kann beispielsweise glatt ausgebildet sein. In manchen Ausführungsformen ist es auch denkbar, dass die Druckstutzen-Innenseite 30i strukturiert ausgebildet ist und beispielsweise Rippen, Furchen, Kanäle oder dergleichen aufweist, um die Strömungseigenschaften des Fluids zu optimieren (nicht gezeigt).

Die Innenseiten 16i,17i und/oder die Druckstutzen-Innenseite 30i und/oder die Saugstutzen-Innenseite 20i können in manchen Ausführungsformen im Bereich des Kanals 12 eine im Wesentlichen glatte Oberfläche aufweisen. Die Oberfläche kann auch eine materialbedingte Rauigkeit besitzen. Die Beschaffenheit der Oberfläche kann den Druckverlauf und den Drehklang beeinflussen.

Der Strömungskanal 50 ist in einem Anwendungszustand von der Gehäusewand 11 zumindest teilweise umgeben. Der Strömungskanal 50 verläuft zumindest abschnittsweise kreisförmig. Der Strömungskanal 50 steht mit dem Einlasskanal 24 und dem Auslasskanal 34 in Verbindung.

Über die Einlassöffnung 22 und den Einlasskanal 24 tritt Atemluft in Achsrichtung in die Lüftereinrichtung 1 ein. Der Einlasskanal 24 verläuft beispielsweise zumindest abschnittsweise gerade. Von der Einlassöffnung 22 gelangt die Atemluft über den Einlasskanal 24 in das Lüfterrad 5, das die Atemluft über eine Rotationsbewegung in den Strömungskanal 50 transportiert. Über den weitestgehend kreisförmigen Strömungskanal 50 wird die Atemluft in radialer Richtung transportiert und gelangt in den Auslasskanal 34. Über den Auslasskanal 34 und die Auslassöffnung 32 tritt Atemluft senkrecht zur Achsrichtung aus der Lüftereinrichtung 1 heraus. Dadurch ergibt sich eine spiralförmige Strömungsrichtung 80.

Dort, wo im Inneren des Gehäuses 10 die Gehäusewand 11 und die Druckstutzenwand 31 aufeinandertreffen, ist eine Gehäusezunge 37 ausgebildet. Definitionsgemäß beginnt der Druckstutzen 30 auf Höhe der Gehäusezunge 37 (siehe Fig. 4A).

Die Atemluft strömt auf die Gehäusezunge 37, wodurch ein Drehklang entstehen kann. Auch durch Wirbelablösungen und/oder turbulente An- und/oder Abströmungen können innerhalb der Lüftereinrichtung 1 Strömungsgeräusche entstehen. Ferner entsteht Drehklang durch die Schaufelelemente 6 des Lüfterrades 5, wenn diese die Atemluft verdrängen.

Die Gehäusezunge 37 bildet eine Hauptquelle des Drehklanges. Die Position der Gehäusezunge 37 und/oder der Abstand zwischen Lüfterrad 5 und Gehäusezunge 37 können hierbei einen Einfluss auf die Lautstärke des Drehklanges haben.

Erfindungsgemäß weist das Gehäuse 10 wenigstens ein Strukturelement 33, 60 auf, das die Schallemission verringert.

Der Auslasskanal 34 ist in einem Anwendungszustand von der Druckstutzenwand 31 zumindest teilweise umgeben. Der Auslasskanal 34 verläuft beispielsweise zumindest abschnittsweise gerade.

Der Auslasskanal 34 hat eine Höhe 34H. Üblicherweise ist der Auslasskanal 34 als ein durchgehender Kanal mit einer Höhe 34H ausgebildet (siehe Fig. 4A).

In einer vorteilhaften Ausführungsform kann zumindest der Auslasskanal 34 durch mindestens ein Strukturelement 33 zumindest abschnittsweise in unabhängig voneinander verlaufende Kanäle 35, 36 aufgetrennt sein (siehe Fig. 4B). Die Kanäle 35, 36 werden hierin weiter unten ausführlicher beschrieben. In alternativen Ausführungsformen kann der Auslasskanal 34 auch durch mindestens ein Strukturelement 33 in miteinander in Verbindung stehende Kanäle aufgetrennt sein.

In einer erfindungsgemäßen Ausführungsform kann das Gehäuse 10 der Lüftereinrichtung 1 mindestens ein Strukturelement 33 umfassen. Das Strukturelement kann in Form einer Trennwand 33 ausgebildet sein (siehe Fig. 4B und folgende).

Die mindestens eine Trennwand 33 kann aus demselben Material gefertigt sein, wie die Lüftereinrichtung 1. In manchen Ausführungsformen kann die Trennwand 33 auch aus einem anderen geeigneten Material gefertigt sein. Beispielsweise kann die Trennwand 33 aus Metall oder Kunststoff gefertigt sein. Die Trennwand 33 kann am Vorderteil 16 und/oder am Hinterteil 17 angeformt sein.

Die Trennwand 33 ist mindestens abschnittsweise im Kanal 12 angeordnet. Die Trennwand 33 kann zumindest abschnittsweise im Auslasskanal 34 angeordnet sein. Alternativ oder ergänzend kann die Trennwand 33 im Strömungskanal 50 angeordnet sein.

Die Trennwand 33 kann einteilig oder zweigeteilt ausgebildet sein. In manchen Ausführungsformen kann die Trennwand 33 zweigeteilt ausgebildet sein und sowohl am Hinterteil 17 als auch am Vorderteil 16 angeformt sein. In einem Anwendungszustand kann die zweigeteilte Trennwand 33 zu einer den Kanal 12 zumindest teilweise vollständig trennenden Trennwand 33 zusammengefügt werden. In manchen Ausführungsformen kann die Trennwand 33 den Kanal 12 zumindest abschnittsweise nur unvollständig teilen (nicht gezeigt).

In einer bevorzugten Ausführungsform ist die Trennwand 33 einteilig ausgebildet. In einem Anwendungszustand, also wenn Vorderteil 16 und Hinterteil 17 miteinander verbunden sind, kann die einteilig ausgebildete Trennwand 33 den Kanal 12 zumindest abschnittsweise vollständig teilen. Der Kanal 12 kann durch die Trennwand 33 zumindest abschnittsweise zweigeteilt verlaufen.

Die Trennwand 33 kann beispielsweise einteilig sein und an dem Hinterteil 17 angeformt sein. In diesem Falle kann das Vorderteil 16 eine nicht gezeigte Aufnahmefuge 44 aufweisen, in die die Trennwand 33 eingefügt werden kann. Alternativ kann eine einteilige Trennwand 33 auch an dem Vorderteil 16 angeformt sein. Sodann kann das Hinterteil 17 eine Aufnahmefuge 44 aufweisen, in die die Trennwand 33 eingefügt werden kann (nicht gezeigt).

Die Trennwand 33 kann zumindest abschnittsweise den Kanal 12 in Strömungsrichtung 80 durchlaufen. Die Trennwand 33 hat einen Anfang 38 und ein Ende 45. Der Anfang 38 der Trennwand 33 liegt in Bezug zur Strömungsrichtung weiter vorne und das Ende 45 der Trennwand 33 liegt in Bezug zur Strömungsrichtung 80 weiter hinten.

In manchen Ausführungsformen kann das Ende 45 innerhalb des Gehäuses angeordnet sein (nicht gezeigt). In den hier dargestellten beispielhaften Ausführungsformen kann das Ende 45 direkt auf Höhe der Auslassöffnung 32 angeordnet sein (siehe beispielsweise Fig. 4B).

Der Anfang 38 liegt innerhalb des Gehäuses. Der Anfang 38 der Trennwand 33 ist im Kanal 12 angeordnet. Durch den Anfang der Trennwand 33 wird eine zweite Zunge 38 in das Gehäuse 10 eingeführt. Die zweite Zunge 38 ist innerhalb des Gehäuses angeordnet. Die zweite Zunge 38 entsteht am Anfang der Trennwand 33. Der Anfang der Trennwand 33 und somit die zweite Zunge 38 kann gerade, abgerundet und/oder angeschrägt ausgebildet sein.

Durch die zweite Zunge 38 kann die Amplitude des Drehklangs gemindert werden. Durch die zweite Zunge 38 kann sich der Drehklang auf mehrere Frequenzen aufteilen. Somit kann sich der vom Anwender/Patienten subjektiv wahrgenommene Klang verbessern, da die Tonalität des Drehklangs weniger wird. Die Druckkurve wird durch Einführung einer zweiten Zunge 38 hingegen nicht beeinflusst.

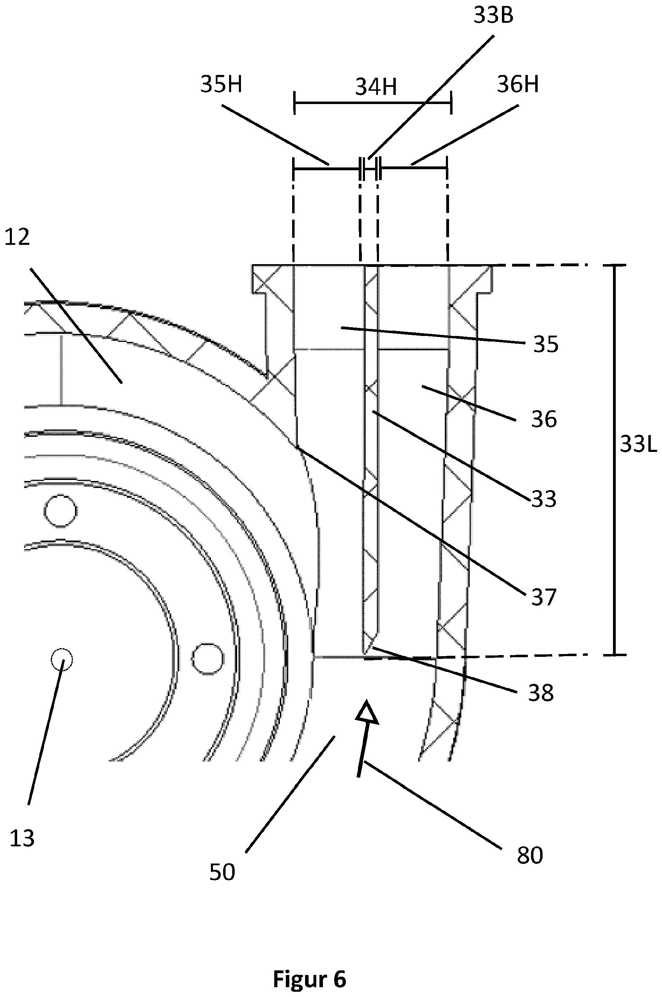

**Figur 6** zeigt einen vergrößerten Ausschnitt aus Fig. 4B. Die Trennwand 33 kann den Kanal 12 zumindest abschnittsweise entlang der Strömungsrichtung 80 teilen. Die Trennwand 33 weist eine Länge 33L auf. Die Länge 33L kann unterschiedlich lang ausgebildet sein. Je nach Länge 33L kann die Trennwand 33 den Strömungskanal 50 und/oder den Auslasskanal 34 zumindest abschnittsweise entlang der Strömungsrichtung 80 teilen.

Die Länge der Trennwand 33L kann derart gewählt sein, dass 5% bis 100% des Auslasskanals 34 durch die Trennwand 33 geteilt wird. Bevorzugt wird 20% bis 100% des Auslasskanals 34 durch die Trennwand 33 geteilt, besonders bevorzugt wird 50% bis 100% des Auslasskanals 34 durch die Trennwand 33 geteilt.

In einem konkreten Ausführungsbeispiel ist die Länge der Trennwand 33L derart eingerichtet und ausgebildet, dass die Trennwand 33 den Auslasskanal 34 der Länge nach vollständig durchläuft. 100% des Auslasskanals 34 sind durch die Trennwand 33 geteilt (siehe Fig. 6). Die Trennwand 33 kann alternativ oder zusätzlich auch den Strömungskanal 50 zumindest teilweise durchlaufen.

Die Länge der Trennwand 33L kann derart gewählt sein, dass 0% bis 90% des Strömungskanal 50 durch die Trennwand 33 geteilt wird. Bevorzugt wird 5% bis 80% des Strömungskanal 50 durch die Trennwand 33 geteilt.

Die Trennwand 33 kann sich in dem in Fig. 6 dargestellten konkreten Ausführungsbeispiel bis auf Höhe der Mittelachse 13 erstrecken. Sodann durchläuft die Trennwand 33 den Auslasskanal 34 sowie zumindest teilweise den Strömungskanal 50. In dem in Fig. 6 dargestellten konkreten Ausführungsbeispiel wird 100% des Auslasskanals 34 und zusätzlich ca. 15% des Strömungskanals 50 durch die Trennwand 33 geteilt.

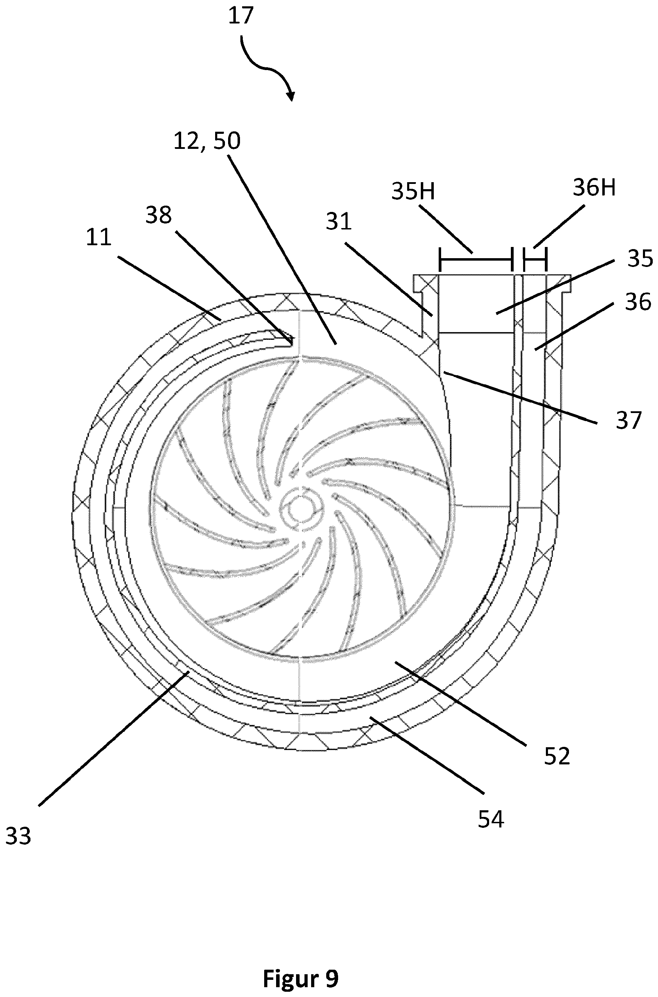

Die Trennwand 33 kann sich in dem in Fig. 6 dargestellten konkreten Ausführungsbeispiel bis zur Auslassöffnung 32 erstrecken. In manchen Ausführungsformen kann die Trennwand 33 auch kürzer ausgebildet sein und innerhalb des Auslasskanals 34 und/ oder innerhalb des Strömungskanals 50 enden (nicht gezeigt). In alternativen Ausführungsbeispielen kann die Trennwand 33 auch länger (siehe Fig.9-10) oder kürzer (nicht gezeigt) ausgebildet sein.

Die Trennwand 33 weist eine Breite 33B auf. Die Breite der Trennwand 33B kann ebenso breit sein oder breiter sein als die Gehäusewand 11. In einer bevorzugten Ausführungsform ist die Breite der Trennwand 33B schmaler ausgebildet als die Gehäusewand 11. Die Breite der Trennwand 33B liegt in einem Bereich von 0,1 mm bis 8 mm, bevorzugt in einem Bereich von 0,5 mm bis 5 mm, besonders bevorzugt in einem Bereich von 0,5 mm bis 2 mm. Beispielsweise beträgt die Breite der Trennwand 33B 1 mm. Die Trennwand 33 sollte so dünn wie möglich ausgebildet sein, um den Strömungsquerschnitt der Kanäle möglichst groß zu halten. Die Trennwand 33 sollte dennoch so stark ausgebildet sein, dass sie eine ausreichende Stabilität besitzt.

Die Trennwand 33 kann den Auslasskanal 34 derart trennen, dass ein innerer Auslasskanal 35 mit einer Höhe 35H und ein äußerer Auslasskanal 36 mit einer Höhe 36H entsteht. Über den äußeren Auslasskanal 36 kann die Atemluft aus dem Gehäuse heraustransportiert werden. Über den inneren Auslasskanal 35 kann die Atemluft aus dem Gehäuse 10 heraustransportiert werden und/oder erneut in den Strömungskanal 50 gelangen. Die Anordnung der Trennwand 33 im Kanal 12 kann einen Einfluss auf das Geschwindigkeitsprofil und/oder auf die Druckschwankungen der Strömung haben.

Die Trennwand 33 kann mittig im Auslasskanal 34 angeordnet sein. Bei einer mittigen Anordnung ist der Auslasskanal zweigeteilt und die Höhe 35H des inneren Auslasskanals 35 entspricht der Höhe 36H des äußeren Auslasskanals 36 (siehe Fig. 6).

In alternativen Ausführungsformen kann die Höhe 35H des inneren Auslasskanals 35 auch kleiner als die Höhe 36H des äußeren Auslasskanals 36 ausgebildet sein. In manchen Ausführungsformen kann die Höhe 35H des inneren Auslasskanals 35 auch größer als die Höhe 36H des äußeren Auslasskanals 36 ausgebildet sein (nicht gezeigt).

Die Lage des Laufrades 5 zur Trennwand 33 wird durch die radiale Anordnung der Trennwand innerhalb des Kanals 12 bedingt. Ist die Trennwand 33 radial weiter nach außen angeordnet, vergrößert sich der Abstand zum Laufrad 5. Ist die Trennwand 33 radial weiter nach innen angeordnet, verkleinert sich der Abstand zum Laufrad 5. Ein großer Abstand der Trennwand 33 zum Laufrad 5 kann vorteilhaft sein, da dadurch das Geschwindigkeitsprofil geglättet werden kann und Druckschwankungen an der Zunge 37 verringert werden können.

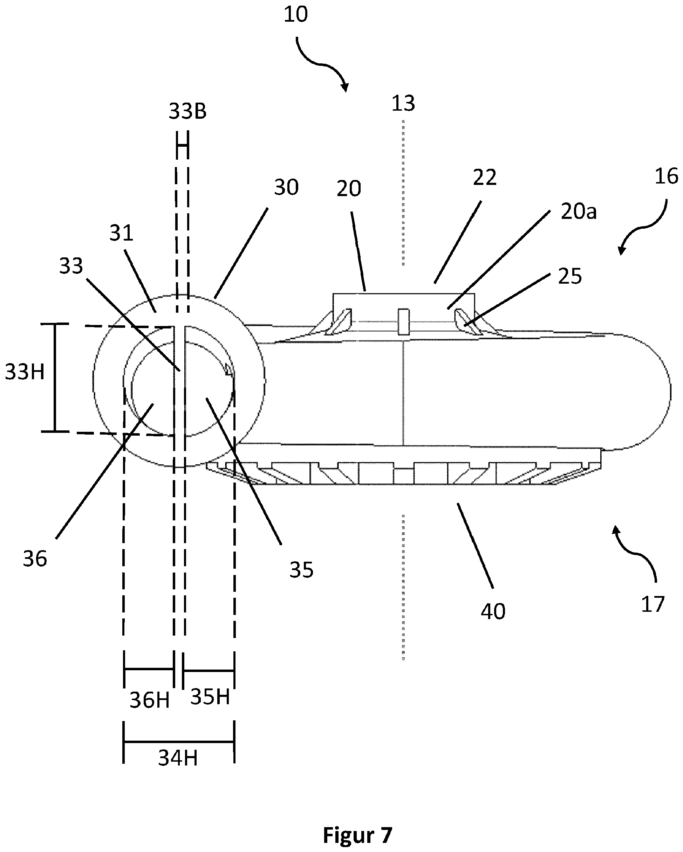

**Figur 7** eine schematische Außenansicht des Gehäuses 10 aus Fig. 4-6 von der Seite mit Blickrichtung in einen Druckstutzen 30 hinein.

Die Trennwand 33 weist eine Höhe 33H auf. Die Trennwand 33 kann zwischen Vorderteil 16 und Hinterteil 17 durchgehend ausgebildet sein. In dem Falle steht die Trennwand 33 mit der Innenseite des Vorderteils 16i und mit der Innenseite des Hinterteils 17i in Verbindung. So kann die Trennwand 33 den Kanal 12 zumindest abschnittsweise vollständig teilen. Beispielsweise trennt die Trennwand 33 den Auslasskanal 34 vollständig in zwei unabhängig voneinander verlaufende Kanäle 35, 36 (Fig.7).

In manchen Ausführungsformen kann die Höhe der Trennwand 33H auch kleiner ausgebildet sein, als der Durchmesser des Auslasskanals 34. Dann kann die Trennwand 33 nicht durchgehend ausgebildet sein und nur mit der Innenseite des Vorderteils 16i oder nur mit der Innenseite des Hinterteils 17i verbunden sein. So kann die Trennwand 33 den Kanal 12 zumindest abschnittsweise unvollständig teilen (nicht gezeigt).

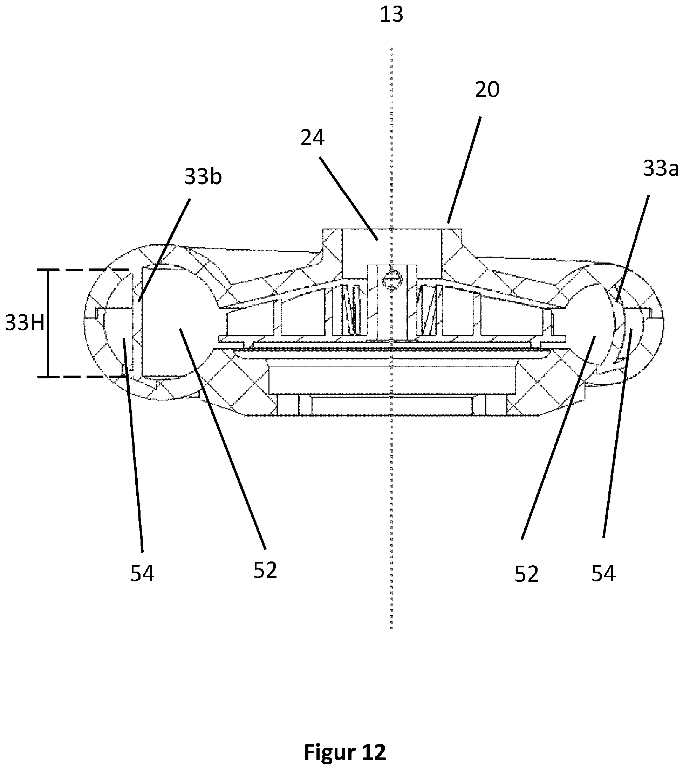

Die Trennwand 33 kann entlang der Höhe 33H gerade ausgebildet sein (Fig. 7). In manchen Ausführungsformen kann die Trennwand 33 entlang der Höhe 33H auch konvex und/oder konkav gebogen ausgebildet sein (siehe Fig. 12).

**Figur 8** zeigt einen Querschnitt durch das Gehäuse 10 aus Fig. 4-7. Aus Fig. 8 wird ersichtlich, dass der Saugstutzen 20 aus der Außenkontur des Gehäuses 10 hervorragt. Die Saugstutzen-Innenseite 20i umfasst das Lumen des Saugstutzens 20, in dem der Einlasskanal 24 verläuft. Über das Lumen des Saugstutzens 20 tritt ein Fluid, beispielsweise Atemluft, in das Gehäuse 10 ein. Die Strömungsrichtung 80 in das Gehäuse 10 hinein ist mit einem Pfeil gekennzeichnet. Die Strömungsrichtung 80 verläuft vom Einlasskanal 24 des Saugstutzens 20 in das Laufrad 5 und vom Laufrad 5 über den Strömungskanal 50 in den Auslasskanal 34 (hier nicht dargestellt).

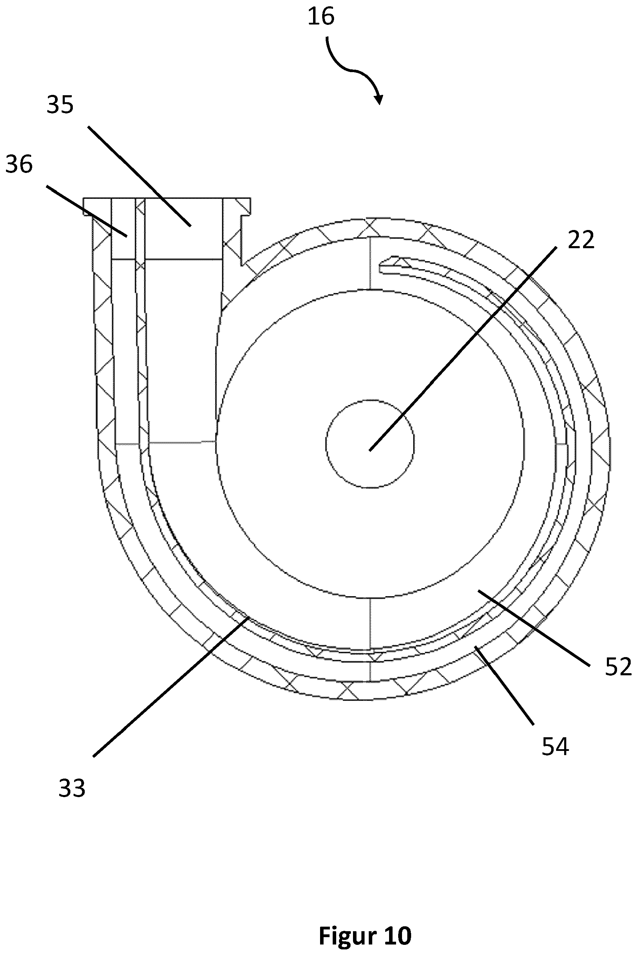

**Figur 9** zeigt einen Längsschnitt durch ein erfindungsgemäßes Gehäuse 10 eines weiteren Ausführungsbeispiels mit einer Innenansicht des Hinterteils 17 mit eingesetztem Lüfterrad und **Figur 10** zeigt einen Längsschnitt durch das Gehäuse 10 des weiteren Ausführungsbeispiels mit einer Innenansicht des Vorderteils 16.

In dem konkreten Ausführungsbeispiel nach Fig. 9/10 ist die Trennwand 33 wesentlich länger ausgebildet als zuvor beschrieben. In diesem konkreten Ausführungsbeispiel ist die Länge der Trennwand 33L derart eingerichtet und ausgebildet, dass die Trennwand 33 den Auslasskanal 34 der Länge nach vollständig durchläuft. Der Auslasskanal 34 wird durch die Trennwand 33 unterteilt in einen inneren Auslasskanal 35 und einen äußeren Auslasskanal 36. Beispielsweise sind 100% des Auslasskanals 34 durch die Trennwand 33 geteilt.

Zusätzlich kann die Trennwand 33 zumindest abschnittsweise auch im Strömungskanal 50 angeordnet sein. Der Strömungskanal 50 kann durch die Trennwand 33 in einen inneren Strömungskanal 52 und einen äußeren Strömungskanal 54 unterteilt sein.

In diesem Ausführungsbeispiel wird 100% des Auslasskanals 34 und zusätzlich ca. 80 % des Strömungskanals 50 durch die Trennwand 33 geteilt. Somit kann die spiralförmige Strömungsrichtung 80 weitestgehend zweigeteilt verlaufen. Dadurch, dass die Trennwand 33 derart lang ausgebildet ist, dass sie zusätzlich im Strömungskanal 50 angeordnet ist, ist die zweite Zunge 38 weit innerhalb des Gehäuses 10 angeordnet. Die zweite Zunge 38 befindet sich am Beginn der Strömungsrichtung 80. Das bietet den Vorteil, dass eine bessere Strömungsführung in der Spirale gegeben ist und/oder weniger Querbewegungen des Fluids möglich sind.

In manchen Ausführungsformen kann die Trennwand 33 auch ausschließlich im Strömungskanal 50 angeordnet sein und den Strömungskanal 50 zumindest teilweise teilen (nicht gezeigt).

Die Trennwand 33 kann in manchen Ausführungsformen immer mittig im Kanal 12 angeordnet sein (nicht gezeigt). Die Anordnung der Trennwand 33 innerhalb des Kanals 12 kann sich mit dem Verlauf der Strömungsrichtung 80 verändern (siehe beispielsweise Fig. 9). In dem hier dargestellten konkreten Ausführungsbeispiel ist die Trennwand 33 derart angeordnet, dass der äußere Strömungskanal 54 in seiner Breite nahezu gleichbleibt, wohingegen der innere Strömungskanal 52 in seiner Breite sukzessive zunimmt. Dementsprechend ist die Trennwand im Auslasskanal nicht mittig angeordnet, sondern radial nach außen verlagert. Somit kann die Höhe des äußeren Auslasskanals 36H kleiner als die Höhe des inneren Auslasskanals 35H ausgebildet sein.

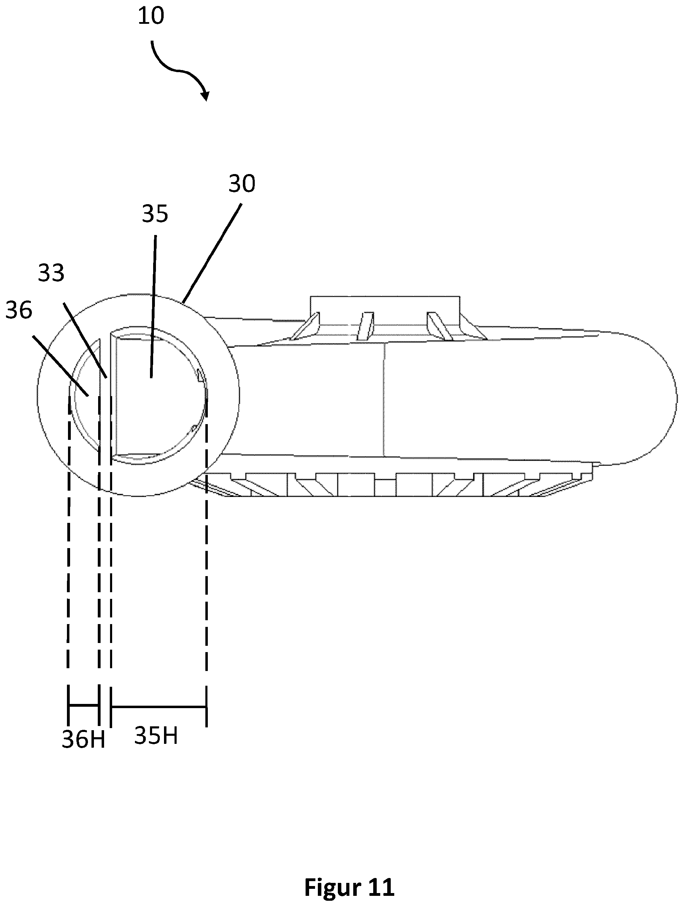

**Figur 11** zeigt eine schematische Außenansicht des Gehäuses 10 entsprechend des weiteren Ausführungsbeispiels aus Fig. 9-10 von der Seite mit Blickrichtung in einen Druckstutzen 30 hinein. Aus Fig. 11 wird ersichtlich, dass die Trennwand 33 im Druckstutzen 30 nicht mittig angeordnet sein muss. Die Trennwand 33 ist in diesem Ausführungsbeispiel radial nach außen angeordnet. Dadurch ist die Höhe des inneren Auslasskanals 35H größer als die Höhe des äußeren Auslasskanals 36H. In alternativen Ausführungsformen kann die Trennwand 33 auch mittig angeordnet sein. Dann kann die Höhe des inneren Auslasskanals 35H gleich der Höhe des äußeren Auslasskanals 36H sein (siehe Fig. 7). In manchen Ausführungsformen kann die Trennwand 33 auch radial nach innen angeordnet sein. Dann kann die Höhe des inneren Auslasskanals 35H kleiner als die Höhe des äußeren Auslasskanals 36H sein (nicht gezeigt). Aus Fig. 7 wird zudem ersichtlich, dass die Trennwand 33 im Druckstutzen 30 gerade ausgebildet ist. In manchen Ausführungsformen kann die Trennwand 33 alternativ oder zusätzlich zumindest abschnittsweise auch konvex und/oder konkav gebogen ausgebildet sein (siehe Fig. 12).

**Figur 12** zeigt einen Querschnitt durch das Gehäuse 10 des weiteren Ausführungsbeispiels aus Fig. 9-11. Aus Fig. 12 wird ersichtlich, dass die Trennwand 33 auch im Strömungskanal 50 angeordnet ist. Durch die Anordnung der Trennwand 33 im Strömungskanal 50 ist dieser in einen inneren Strömungskanal 52 und einen äußeren Strömungskanal 54 getrennt. In diesem konkreten Ausführungsbeispiel ist die Trennwand 33 nicht mittig im Strömungskanal 50 angeordnet, sondern zunehmend radial nach außen verlagert. Dadurch kann der innere Strömungskanal 52 größer ausgebildet sein als der äußere Strömungskanal 54. In manchen Ausführungsformen kann die Trennwand 33 auch derart im Strömungskanal 50 angeordnet sein, dass innerer Strömungskanal 52 und äußerer Strömungskanal 54 gleich groß ausgebildet sind und/oder derart, dass der innere Strömungskanal 52 kleiner als der äußere Strömungskanal 54 ausgebildet ist (nicht gezeigt). Das Verhältnis der Größe des inneren Strömungskanals 52 und des äußeren Strömungskanals 54 kann im Laufe der Strömungsrichtung 80 gleichbleiben (nicht gezeigt) oder sich zueinander verändern (Fig. 9-12).

Aus Fig. 12 wird darüber hinaus ersichtlich, dass die Trennwand 33 entlang der Höhe 33H gerade und/oder gebogen ausgebildet sein kann. In diesem konkreten Ausführungsbeispiel ist die Trennwand 33 zunächst gebogen ausgebildet (33a) und verändert seine Ausbildung im weiteren Strömungsverlauf derart, dass die Trennwand 33 gerade ausgebildet ist (33b).

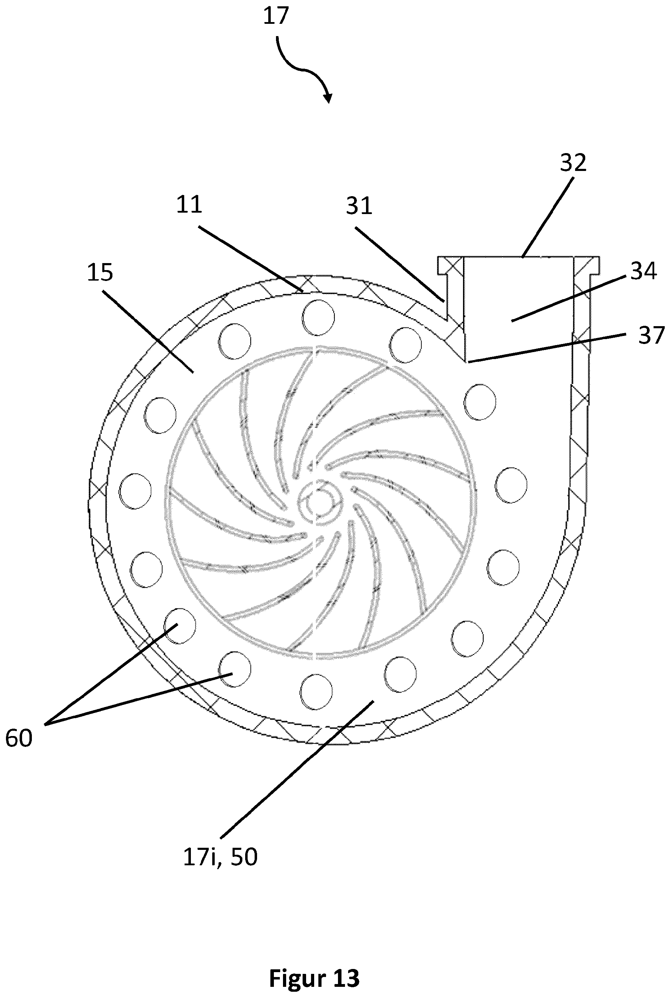

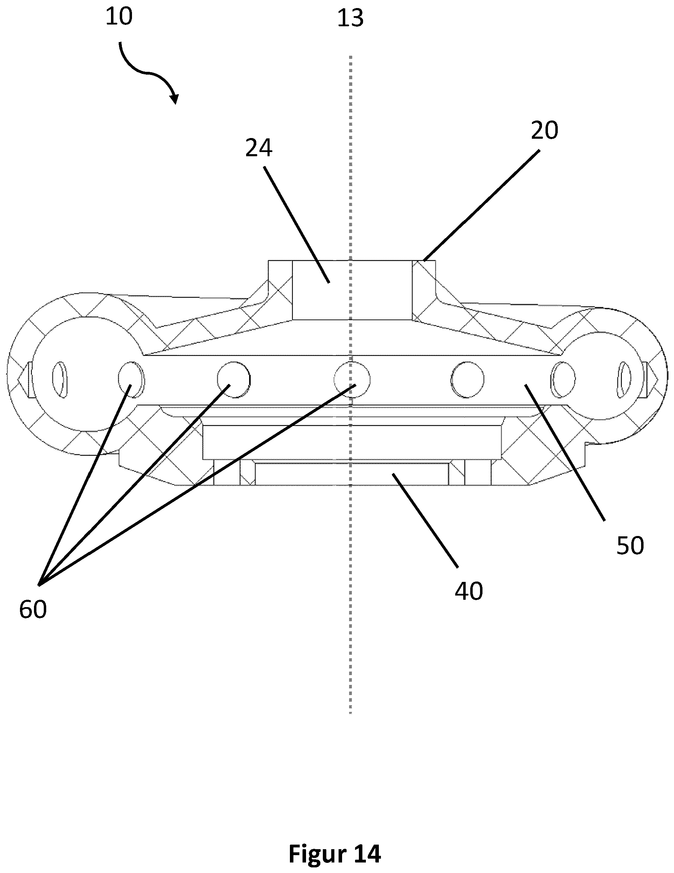

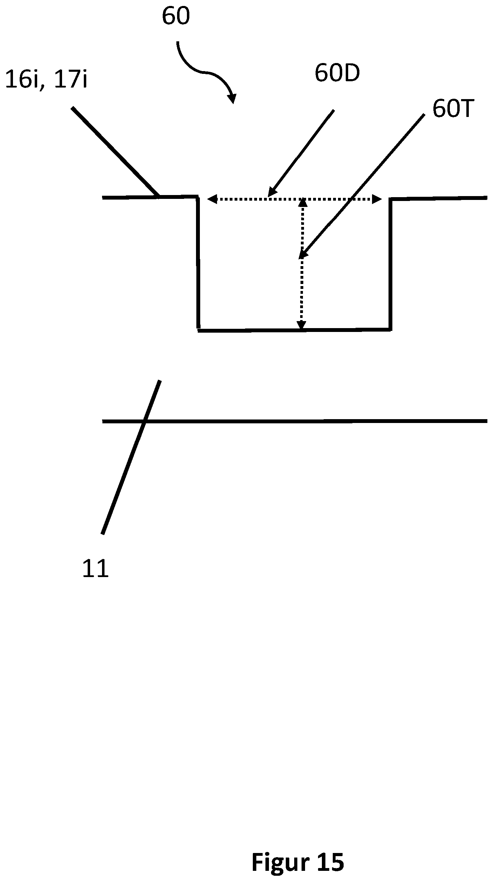

**Figur 13** zeigt einen Längsschnitt durch ein erfindungsgemäßes Gehäuse 10 eines alternativen Ausführungsbeispiels mit einer Innenansicht des Hinterteils 17 mit eingesetztem Lüfterrad 5. **Figur 14** zeigt einen Querschnitt durch das Gehäuse 10 des alternativen Ausführungsbeispiels aus Fig. 13. **Figur 15** zeigt schematisch einen Querschnitt durch die Gehäusewand 11 einer Innenseite des Vorderteils 16i bzw. einer Innenseite des Hinterteils 17i des alternativen Ausführungsbeispiels aus Fig. 13/14.

In Fig. 13 bis 15 ist eine weitere erfindungsgemäße Ausführungsform gezeigt, bei der das Gehäuse 10 der Lüftereinrichtung 1 alternativ oder zusätzlich mindestens ein Strukturelement 60 umfassen kann. Das Strukturelement kann in Form mindestens einer Senkbohrung 60 ausgebildet sein. Die eine oder mehrere Senkbohrungen 60 können alternativ oder zusätzlich zu der Trennwand 33 in dem Gehäuse 10 angeordnet sein.

Die Senkbohrungen 60 können bevorzugt am Strömungskanal 50 und/oder am Auslasskanal 34 angeordnet sein. Die Senkbohrungen 60 können in oder an der Innenseite 16i, 17i im Bereich des Strömungskanals 50 und/oder des Auslasskanals 34 angeordnet sein. Durch die Senkbohrungen 60 kann eine Struktur in die innere Oberfläche 16i, 17i eingeführt werden, die sich in Bezug auf den Drehklang positiv auswirken kann.

Die Senkbohrungen 60 können an der Innenseite des Hinterteils 17i (Fig. 13) und/oder an der Innenseite des Vorderteils 16i (nicht gezeigt) angeordnet sein. Die Senkbohrungen 60 können beispielsweise durch eine Vertiefung in der Innenseite des Hinterteils 17i und/oder in der Innenseite des Vorderteils 16i ausgebildet sein. Die Senkbohrungen 60 haben eine Tiefe 60T (siehe Fig. 15). Die Senkbohrungen 60 sind wenigstens auf einer der Innenseiten des Gehäuses 16i, 17i angeordnet, bevorzugt auf beiden Innenseiten 16i, 17i. Die Senkbohrungen 60 sind derart eingerichtet und ausgebildet, dass die ansonsten im Wesentlichen glatte Oberfläche der Innenseiten 16i, 17i Abschnitte aufweisen, die vertieft vorliegen.

Die Senkbohrungen 60 können gleichmäßig verteilt sein. In manchen Ausführungsformen können die Senkbohrungen 60 nur in bestimmten Bereichen oder Abschnitten punktuell angeordnet sein (nicht gezeigt). Die Senkbohrungen 60 können auch zufällig verteilt über die Innenseiten 16i und/oder 17 i angeordnet sein.

Die Senkbohrungen 60 können eine Tiefe 60T von 0,5 mm bis 2,5 mm aufweisen. Bevorzugt können die Senkbohrungen 60 eine Tiefe 60T von 1 mm bis 2 mm aufweisen. Beispielsweise weisen die Senkbohrungen 60 eine Tiefe 60T von 1,4 mm auf. Der Grad der Vertiefung ist derart gewählt, dass die Tonalität verringert wird. Der Grad der Vertiefung ist derart gewählt, dass der Gesamtschalldruck verringert wird oder gleichbleibt. Der Grad der Vertiefung ist derart gewählt, dass die Kennlinien verbessert werden können. Die Senkbohrungen 60 können alle gleich tief ausgebildet sein oder in ihrer Tiefe 60T variieren. Die Senkbohrungen 60 können in manchen Ausführungsformen an speziellen Bereichen der Innenseiten 16i, 17i abweichende Tiefen 60T aufweisen.

Die Vertiefung der Senkbohrungen 60 kann gerade verlaufen (siehe Fig. 15). In manchen Ausführungsformen kann die Vertiefung der Senkbohrungen 60 sich konisch verengen und/oder aufweiten (nicht gezeigt). Die Senkbohrungen 60 können in manchen Ausführungsformen eine zufällige Form aufweisen.

Die Senkbohrungen 60 können jegliche geeignete Geometrie aufweisen. In einer bevorzugten Ausführungsform können die Senkbohrungen 60 rund und/oder oval ausgebildet sein. Die Senkbohrungen 60 haben einen Durchmesser 60D (siehe Fig. 15). Die Senkbohrungen 60 können einen Durchmesser 60D von 1 mm bis 7 mm aufweisen. Bevorzugt weisen die Senkbohrungen 60 einen Durchmesser 60D von 2 mm bis 5 mm auf. Beispielsweise können die Senkbohrungen 60 einen Durchmesser 60D von 4 mm aufweisen.

Die Innenseite des Hinterteils 17i und/oder die Innenseite des Vorderteils 16i können bis zu 100 oder mehr Senkbohrungen 60 aufweisen. Die Anzahl der Senkbohrungen 60 kann in einem Bereich von 1 bis 100 liegen, bevorzugt in einem Bereich von 1 bis 50, besonders bevorzugt in einem Bereich von 4 bis 20. Beispielsweise können die Innenseite des Hinterteils 17i und/oder die Innenseite des Vorderteils 16i jeweils 12 Senkbohrungen 60 aufweisen. Die Anzahl der Senkbohrungen 60 ist derart gewählt, dass die Tonalität abnimmt. Die Anzahl der Senkbohrungen 60 ist derart gewählt, dass der Gesamtschalldruck verringert wird oder gleichbleibt.

Aus Fig. 14 wird ersichtlich, dass die Senkbohrungen 60 in manchen Ausführungsformen alternativ oder zusätzlich auch in der inneren Umfangswandung des Gehäuses angeordnet sein können. Die Senkbohrungen im Gehäuse bewirken, dass die Schallemission wesentlich verringert wird.

Obwohl die vorliegende Erfindung anhand der Ausführungsbeispiele detailliert beschrieben wurde, ist es für den Fachmann selbstverständlich, dass die Erfindung nicht auf diese Ausführungsbeispiele beschränkt ist. Vielmehr sind Abwandlungen in einer Weise möglich, dass einzelne Merkmale weggelassen werden oder andersartige Kombinationen der beschriebenen Einzelmerkmale verwirklicht werden können, sofern der Schutzbereich der beiliegenden Ansprüche nicht verlassen wird. Die vorliegende Offenbarung schließt sämtliche Kombinationen der vorgestellten Einzelmerkmale mit ein.

### Bezugszeichenliste

| | |
|---|---|
| 1 | Lüftereinrichtung |
| 5 | Lüfterrad |
| 6 | Schaufelelemente |
| 10 | Gehäuse |
| 11 | Gehäusewand |
| 12 | Kanal |
| 13 | Mittelachse |
| 16 | Vorderteil |
| 16a | Außenseite des Vorderteils |
| 16i | Innenseite des Vorderteils |
| 17 | Hinterteil |
| 17a | Außenseite des Hinterteils |
| 17i | Innenseite des Hinterteils |
| 18 | Verbindungsfuge |
| 20 | Saugstutzen |
| 20a | Saugstutzen-Außenseite |
| 20i | Saugstutzen-Innenseite |
| 21 | Saugstutzenwand |
| 22 | Einlassöffnung |
| 24 | Einlasskanal |
| 25 | Stützrippen |
| 29 | Saugstutzen-Flansch |
| 30 | Druckstutzen |
| 30a | Druckstutzen-Außenseite |
| 30i | Druckstutzen-Innenseite |
| 31 | Druckstutzenwand |
| 32 | Auslassöffnung |
| 33 | Trennwand |
| 33B | Breite der Trennwand |
| 33H | Höhe der Trennwand |
| 33L | Länge der Trennwand |
| 34 | Auslasskanal |
| 34H | Höhe des Auslasskanals |
| 35 | Innerer Auslasskanal |
| 35H | Höhe des inneren Auslasskanals |
| 36 | Äußerer Auslasskanal |
| 36H | Höhe des äußeren Auslasskanals |
| 37 | Gehäusezunge |
| 38 | Zweite Zunge |
| 39 | Drucksstutzen-Flansch |
| 40 | Öffnung |
| 41 | Elemente |
| 42 | Bohrung |
| 43 | Aufnahmeeinrichtung |
| 44 | Aufnahmefuge |
| 45 | Ende |
| 50 | Strömungskanal |
| 52 | Innerer Strömungskanal |
| 54 | Äußerer Strömungskanal |
| 60 | Senkbohrung |
| 60D | Durchmesser der Senkbohrung |
| 60T | Tiefe der Senkbohrung |
| 70 | Atemtherapiegerät |
| 71 | Bedieneinrichtung |
| 72 | Anzeigeeinrichtung |
| 73 | Schlauchsystem |
| 74 | Steuereinrichtung |
| 75 | Patienteninterface |
| 76 | Schnittstelle |
| 80 | Strömungsrichtung |

## Patentansprüche

1. Atemtherapiegerät (70) mit wenigstens einer Lüftereinrichtung (1) zum Erzeugen einer Atemluftströmung für die Durchführung einer Atemtherapie, wobei die Lüftereinrichtung (1) ein Gehäuse (10) und wenigstens ein in dem Gehäuse (10) drehbar gelagertes Lüfterrad (5) umfasst, wobei die Atemluft durch einen innerhalb des Gehäuses (10) ausgebildeten Kanal (12) transportiert wird, wobei das Gehäuse (10) wenigstens ein Strukturelement (60) aufweist, das die Schallemission verringert, wobei das Gehäuse (10) mindestens eine Innenseite (16i, 17i) umfasst und der Kanal (12) ein Lumen umfasst, wobei das Lumen des Kanals (12) von der Innenseite (16i und/oder 17i) umschlossen ist, **dadurch gekennzeichnet, dass** das mindestens eine Strukturelement (60) in der Innenseite (16i, 17i) in Form einer Vertiefung in der ansonsten im Wesentlichen glatten Oberfläche der Innenseite (16i, 17i) ausgebildet ist.

2. Atemtherapiegerät (70) nach zumindest einem der vorhergehenden Ansprüche, **dadurch gekennzeichnet, dass** die Innenseite (16i, 17i) 1 bis 100 Vertiefungen aufweisen, bevorzugt 1 bis 50, besonders bevorzugt 4 bis 20.

3. Atemtherapiegerät (70) nach zumindest einem der vorhergehenden Ansprüche, **dadurch gekennzeichnet, dass** die Vertiefungen eine Tiefe (60T) von 0,5 mm bis 2,5 mm, bevorzugt von 1 mm bis 2 mm, besonders bevorzugt von 1,4 mm aufweisen.

4. Atemtherapiegerät (70) nach zumindest einem der vorhergehenden Ansprüche, **dadurch gekennzeichnet, dass** die Vertiefungen rund und/oder oval ausgebildet sind.

5. Atemtherapiegerät (70) nach zumindest einem der vorhergehenden Ansprüche, **dadurch gekennzeichnet, dass** die Vertiefungen einen Durchmesser (60D) von 1 mm bis 7 mm, bevorzugt von 2 mm bis 5 mm, besonders bevorzugt von 4 mm aufweisen.

6. Atemtherapiegerät (70) nach zumindest einem der vorhergehenden Ansprüche, **dadurch gekennzeichnet, dass** der Kanal (12) einen Einlasskanal (24), mindestens einen Strömungskanal (50) und mindestens einen Auslasskanal (34) umfasst, die kommunizierend miteinander in Verbindung stehen, wobei der Kanal (12) einen Strömungsweg mit einer Strömungsrichtung (80) ausbildet, wobei die Strömungsrichtung (80) vom Einlasskanal (24) zum Strömungskanal (50) zum Auslasskanal (34) verläuft, wobei die Vertiefungen in der Innenseite (16i, 17i) im Bereich des Strömungskanals (50) und/oder des Auslasskanals (34) angeordnet sind.

7. Atemtherapiegerät (70) nach zumindest einem der vorhergehenden Ansprüche, **dadurch gekennzeichnet, dass** die Vertiefungen in der inneren Umfangswandung des Gehäuses angeordnet sind.

8. Atemtherapiegerät 70 nach zumindest einem der vorhergehenden Ansprüche, **dadurch gekennzeichnet, dass** die Strukturelemente (60) als Senkbohrungen ausgebildet sind.

9. Atemtherapiegerät 70 nach zumindest einem der vorhergehenden Ansprüche, **dadurch gekennzeichnet, dass** das Strukturelement zusätzlich als mindestens eine Trennwand (33) ausgebildet ist, die im Lumen des Kanals 12 angeordnet ist und den Strömungskanal 50 und/oder den Auslasskanal 34 zumindest abschnittsweise zumindest teilweise in mindestens zwei Kanäle 35,36,52,54 unterteilt.

10. Lüftereinrichtung (1) zum Erzeugen einer Atemluftströmung für die Durchführung einer Atemtherapie, wobei die Lüftereinrichtung (1) ein Gehäuse (10) und wenigstens ein in dem Gehäuse (10) drehbar gelagertes Lüfterrad (5) umfasst, wobei die Atemluft durch einen innerhalb des Gehäuses (10) ausgebildeten Kanal (12) transportiert wird, wobei das Gehäuse (10) wenigstens ein Strukturelement (60) aufweist, das die Schallemission verringert, wobei das Gehäuse (10) mindestens eine Innenseite (16i, 17i) umfasst und der Kanal (12) ein Lumen umfasst, wobei das Lumen des Kanals (12) von der Innenseite (16i und/oder 17i) umschlossen ist, **dadurch gekennzeichnet, dass** das mindestens eine Strukturelement (60) in der Innenseite (16i, 17i) in Form einer Vertiefung in der ansonsten im Wesentlichen glatten Oberfläche der Innenseite (16i, 17i) ausgebildet ist.

## Claims

1. A breathing therapy device (70) comprising at least one fan apparatus (1) for generating a breathing air flow for performing breathing therapy, wherein the fan apparatus (1) comprises a housing (10) and at least one fan impeller (5) mounted rotatably in the housing (10), wherein the breathing air is transported through a channel (12) formed within the housing (10), wherein the housing (10) has at least one structural element (60) that reduces sound emission, wherein the housing (10) comprises at least one inner side (16i, 17i) and the channel (12) comprises a lumen, wherein the lumen of the channel (12) is enclosed by the inner side (16i and/or 17i), **characterized in that** the at least one structural element (60) is formed in the inner side (16i, 17i) in the form of a recess in the otherwise substantially smooth surface of the inner side (16i, 17i).

2. The breathing therapy device (70) according to at least one of the preceding claims, **characterized in that** the inner sides (16i, 17i) have 1 to 100 recesses, preferably 1 to 50, more preferably 4 to 20.

3. The breathing therapy device (70) according to at least one of the preceding claims, **characterized in that** the recesses have a depth (60T) of 0.5 mm to 2.5 mm, preferably 1 mm to 2 mm, particularly preferably 1.4 mm.

4. The breathing therapy device (70) according to at least one of the preceding claims, **characterized in that** the recesses are designed to be round and/or oval.

5. The breathing therapy device (70) according to at least one of the preceding claims, **characterized in that** the recesses have a diameter (60D) of 1 mm to 7 mm, preferably 2 mm to 5 mm, particularly preferably 4 mm.

6. The breathing therapy device (70) according to at least one of the preceding claims, **characterized in that** the channel (12) comprises an inlet channel (24), at least one flow channel (50), and at least one outlet channel (34) that are connected so as to communicate with one another, wherein the channel (12) forms a flow path having a flow direction (80), wherein the flow direction (80) extends from the inlet channel (24) to the flow channel (50) to the outlet channel (34), wherein the recesses in the inner side (16i, 17i) are disposed in the region of the flow channel (50) and/or outlet channel (34).

7. The breathing therapy device (70) according to at least one of the preceding claims, **characterized in that** the recesses are disposed in the inner circumferential wall of the housing.

8. The breathing therapy device 70 according to at least one of the preceding claims, **characterized in that** the structural elements (60) are designed as countersunk holes.

9. The breathing therapy device 70 according to at least one of the preceding claims, **characterized in that** the structural element is additionally designed as at least one partition wall (33) which is disposed in the lumen of the channel 12 and subdivides the flow channel 50 and/or outlet channel 34 at least in portions at least partially into at least two channels 35, 36, 52, 54.

10. A fan apparatus (1) for generating a breathing air flow for performing breathing therapy, wherein the fan apparatus (1) comprises a housing (10) and at least one fan impeller (5) mounted rotatably in the housing (10), wherein the breathing air is transported through a channel (12) formed within the housing (10), wherein the housing (10) has at least one structural element (60) that reduces sound emission, wherein the housing (10) comprises at least one inner side (16i, 17i) and the channel (12) comprises a lumen, wherein the lumen of the channel (12) is enclosed by the inner side (16i and/or 17i), **characterized in that** the at least one structural element (60) is formed in the inner side (16i, 17i) in the form of a recess in the otherwise substantially smooth surface of the inner side (16i, 17i).

## Revendications

1. Appareil de thérapie respiratoire (70) comprenant au moins un dispositif de ventilation (1) destiné à générer un écoulement d'air respiratoire pour la mise en œuvre d'une thérapie respiratoire, ledit dispositif de ventilation(1) comprenant un boîtier (10) et au moins une roue de ventilateur (5) montée de façon rotative dans le boîtier (10), l'air respiratoire étant transporté au moyen d'un canal (12) formé à l'intérieur du boîtier (10), le boîtier (10) présentant au moins un élément de structure (60) réduisant l'émission sonore, le boîtier (10) comprenant au moins une face intérieure (16i, 17i) et le canal (12) comprenant une lumière, la lumière du canal (12) étant délimitée par la face intérieure (16i et/ou 17i), **caractérisé en ce que** ledit au moins un élément de structure (60) est formé dans la face intérieure (16i, 17i) sous la forme d'un évidement dans la surface par ailleurs essentiellement lisse de la face intérieure (16i, 17i).

2. Appareil de thérapie respiratoire (70) selon l'une au moins des revendications précédentes, **caractérisé en ce que** les faces intérieures (16i, 17i) présentent de 1 à 100 évidements, de préférence de 1 à 50, de façon particulièrement préférée de 4 à 20.

3. Appareil de thérapie respiratoire (70) selon l'une au moins des revendications précédentes, **caractérisé en ce que** les évidements présentent une profondeur (60T) de 0,5 mm à 2,5 mm, de préférence de 1 mm à 2 mm, de façon particulièrement préférée de 1,4 mm.

4. Appareil de thérapie respiratoire (70) selon l'une au moins des revendications précédentes, **caractérisé en ce que** les évidements sont de forme circulaire et/ou ovale.

5. Apparaeil de thérapie respiratoire (70) selon l'une au moins des revendications précédentes, **caractérisé en ce que** les évidements présentent un diamètre (60D) de 1 mm à 7 mm, de préférence de 2 mm à 5 mm, de façon particulièrement préférée de 4 mm.

6. Appareil de thérapie respiratoire (70) selon l'une au moins des revendications précédentes, **caractérisé en ce que** le canal (12) comprend un canal d'admission (24), au moins un canal d'écoulement (50) et au moins un canal de sortie (34), lesquels sont reliés de façon à communiquer entre eux, le canal (12) formant un chemin d'écoulement avec une direction d'écoulement (80), la direction d'écoulement (80) s'étendant du canal d'admission (24) vers le canal d'écoulement (50) puis vers le canal de sortie (34), les évidements dans la face interne (16i, 17i) étant disposés dans la zone du canal d'écoulement (50) et/ou du canal de sortie (34).

7. Appareil de thérapie respiratoire (70) selon l'une au moins des revendications précédentes, **caractérisé en ce que** les évidements sont disposés dans la paroi périphérique interne du boîtier.

8. Appareil de thérapie respiratoire 70 selon l'une au moins des revendications précédentes, **caractérisé en ce que** les éléments de structure (60) sont réalisés sous forme de contre-alésages.

9. Appareil de thérapie respiratoire 70 selon l'une au moins des revendications précédentes, **caractérisé en ce que** l'élément de structure est en outre réalisé sous la forme d'au moins une cloison (33) disposée dans la lumière du canal 12 et subdivisant le canal d'écoulement 50 et/ou le canal de sortie 34, au moins par sections, au moins partiellement, en au moins deux canaux 35,36,52,54.

10. Dispositif de ventilation (1) destiné à générer un écoulement d'air respiratoire pour la mise en œuvre d'une thérapie respiratoire, le dispositif de ventilation (1) comprenant un boîtier (10) et au moins une roue de ventilateur (5) montée de façon rotative dans le boîtier (10), l'air respiratoire étant transporté au moyen d'un canal (12) formé à l'intérieur du boîtier (10), le boîtier (10) présentant au moins un élément de structure (60) réduisant l'émission sonore, le boîtier (10) comprenant au moins une face intérieure (16i, 17i) et le canal (12) comprenant une lumière, la lumière du canal (12) étant délimitée par la face intérieure (16i et/ou 17i), **caractérisé en ce que** ledit au moins un élément de structure (60) est réalisé dans la face intérieure (16i, 17i) sous la forme d'un évidement dans la surface par ailleurs essentiellement lisse de la face intérieure (16i, 17i).
